# EUROPÄISCHE PATENTANMELDUNG

(11) **EP 0 579 916 A2**
(43) Veröffentlichungstag der Anmeldung: **26.01.1994**
(21) Anmeldenummer: 93107528.7
(22) Anmeldetag: 08.05.1993
(51) Int. Cl.: A61M 39/00, A61M 25/00, A61M 5/142

(54) **Medizinische Schlauch-Verbindungsvorrichtung**

(30) Priorität: 09.07.1992 DE 4222501
(71) Anmelder: B. Braun Melsungen AG, D-34209 Melsungen (DE)
(72) Erfinder: Stabl, Irene, W-3501 Körle (DE); Ritter, Volker, W-3508 Melsungen (DE); Schacht, Bodo, W-3509 Malsfeld (DE)
(74) Vertreter: Selting, Günther, Dipl.-Ing.

(57) **Zusammenfassung**

Zum Verbinden des druckseitigen Endes des Pumpenschlauchs (10) einer peristaltischen Pumpe mit dem Anschlußstück (15) eines Anschlußschlauchs (16) wird der Pumpenschlauch (10) auf das Anschlußstück (15) klebemittelfrei aufgeschoben. Im Falle einer Okklusion des Anschlußschlauchs (16) entsteht im Pumpenschlauch (10) ein erhöhter Druck, der durch ein selbstschließendes Überdruckventil (26) abgelassen wird. Der Öffnungsdruck des Überdruckventils (26) ist kleiner als derjenige Druck, bei dem sich der Pumpenschlauch (10) und das Anschlußstück (15) voneinander lösen. Das Überdruckventil (16) wird durch eine Aussparung an einer Stelle des Umfangs eines am Anschlußstück (15) außen umlaufenden Ringes (22) gebildet.

## Beschreibung

Die Erfindung betrifft eine medizinische Schlauch-Verbindungsvorrichtung zum Verbinden des Pumpenschlauchs eines Schlauchsystems für peristaltische Pumpen mit einem Anschlußschlauch, gemäß DE-GM 84 06 203.7.

In der Medizintechnik werden peristaltische Pumpen beispielsweise in Infusionsgeräten verwendet. Für die peristaltische Pumpe, bei der es sich um eine Rollenpumpe oder eine Fingerpumpe handeln kann, benötigt man einen Pumpenschlauch, der fortlaufend zusammengequetscht wird, um die in ihm enthaltende Flüssigkeit vorzutreiben. Bei medizinischen Geräten ist der Pumpenschlauch Bestandteil eines Schlauchsystems, das von einer Flüssigkeitsquelle zum Patienten führt. Dieses Schlauchsystem ist als Einmalsystem ausgebildet, d.h. es wird nur einmal benutzt und anschließend entsorgt, um Infektionen vorzubeugen. Der in diesem Schlauchsystem enthaltene Pumpenschlauch besteht aus einem Material, das weicher ist als die anderen Schläuche des Schlauchsystems. Es ist daher erforderlich, den Pumpenschlauch mit den weiteren Schläuchen zu verbinden. Normalerweise erfolgt die Verbindung des Pumpenschlauchs mit den Anschlußschläuchen durch Verklebung oder festes Verklemmen. Neuere Einmalsysteme sind vielfach aufgrund von unterschiedlichen Materialien und zur Produktionsvereinfachung nicht mehr verklebt, sondern nur noch gesteckt. Hierbei werden die Zugkräfte von Materialverdickungen und durch die Elastizität des Anschlußschlauchs abgefangen. Eine derartige Verbindungstechnik ist aus DE-GM 84 06 203.7 bekannt. Steckbare Schlauchverbindungen unterliegen der Gefahr der Diskonnektion, wenn der hydraulische Druck im Schlauch eine gewisse Größe erreicht. Peristaltische Pumpen, wie sie in der Medizintechnik verwandt werden, können einen Druck von etwa 3 bar und mehr im Leitungssystem aufbringen. Im Falle einer Schlauchokklusion würde sich ein als Einmalset ausgebildetes Schlauchsystem zerlegen, d.h. der Pumpenschlauch würde von dem Anschlußschlauch getrennt werden. Bei einer Schlauchtrennung an der Druckseite der Pumpe ist der Patient in höchstem Maße gefährdet, da dieser Bereich den patientenseitigen Abschnitt des Schlauchsystems bildet. Durch die Öffnung des zum Patienten führenden Anschlußschlauchs kann Luft angesaugt werden oder Blut vom Patienten abfließen. Beides muß unbedingt verhindert werden.

Der Erfindung liegt die Aufgabe zugrunde, eine Schlauch-Verbindungsvorrichtung zum Verbinden eines Pumpenschlauchs mit einem Anschlußschlauch zu schaffen, bei der ohne Verwendung von Klebemittel sichergestellt ist, daß Diskonnektionen vermieden werden.

Die Lösung dieser Aufgabe erfolgt erfindungsgemäß mit den im Patentanspruch 1 angegebenen Merkmalen.

Bei der erfindungsgemäßen Verbindungsvorrichtung enthält die Verbindungsstelle zwischen Pumpenschlauch und Anschlußschlauch ein selbstschließendes Überdruckventil, dessen Öffnungsdruck kleiner ist als derjenige Druck, bei dem sich der Pumpenschlauch und das Anschlußstück voneinander lösen. Im Falle eines zu hohen Innendrucks im Schlauchsystem öffnet das Überdruckventil nach außen, wodurch Flüssigkeit nach außen entweichen kann. Dadurch wird erreicht, daß der Druck nicht weiter ansteigen kann, so daß die Gefahr von Diskonnektionen der verbundenen Schlauchleitungen vermieden wird. Bei Beendigung des Überdrucks schließt das Überdruckventil selbsttätig wieder, so daß keine Luft von außen in das Schlauchsystem eintreten kann. Ein etwaiger Überdruck wird abgebaut, ohne daß die Schlauchverbindung abreißen kann.

Das Überdruckventil wird durch das Zusammenwirken des elastischen Pumpenschlauchs mit dem relativ starren Anschlußstück gebildet. Für die Funktion des Überdruckventils wird somit die Elastizität des Pumpenschlauchs ausgenutzt, so daß für das Überdruckventil keine zusätzlichen Teile erforderlich sind. Es ist lediglich erforderlich das Anschlußstück und/oder den Pumpenschlauch so auszubilden, daß an einer Stelle des Umfangs eine geringere Anpreßkraft existiert, so daß ein Innendruck im Schlauchsystem an dieser Stelle zuerst entweichen kann, bevor an den übrigen Stellen des Umfangs, an denen der Pumpenschlauch gegen das Anschlußstück gedrückt wird, eine Ablösung erfolgt. Es ist also eine Stelle verringerten Anpreßdrucks am Umfang vorgesehen. Diese Stelle kann dadurch erzeugt werden, daß ein am Anschlußstück vorgesehener Ring oder Wulst an einer Stelle eine Aussparung aufweist, oder dadurch, daß eine entsprechende Aussparung am Innenumfang des Pumpenschlauchs vorgesehen ist. Der Auslösedruck, bei dem das Überdruckventil öffnet, wird hierbei durch den Anpreßdruck des Pumpenschlauchs im Bereich der Aussparung gegen das Anschlußstück bestimmt, während derjenige Druck, bei dem sich der Pumpenschlauch und das Anschlußstück voneinander lösen, durch den höheren Anpreßdruck im übrigen Umfangsbereich bestimmt wird.

Im folgenden werden unter Bezugnahme auf die Zeichnungen Ausführungsbeispiele der Erfindung näher erläutert.

Es zeigen:
- Fig. 1: eine Ansicht eines Teils eines Schlauchsets mit einem Pumpenschlauch und den zugehörigen Anschlußschläuchen,
- Fig. 2: einen Schnitt entlang der Linie II-II von Fig. 1 in vergrößertem Maßstab,
- Fig. 3: eine Stirnansicht des Anschlußstücks aus Richtung des Pfeiles III von Fig. 2,
- Fig. 4: in gleicher Darstellung wie Fig. 3 eine andere Ausführungsform der Aussparung des Anschlußstücks und
- Fig. 5: in gleicher Darstellung wie Fig. 3 eine dritte Ausführungsform der Aussparung des Anschlußstücks.

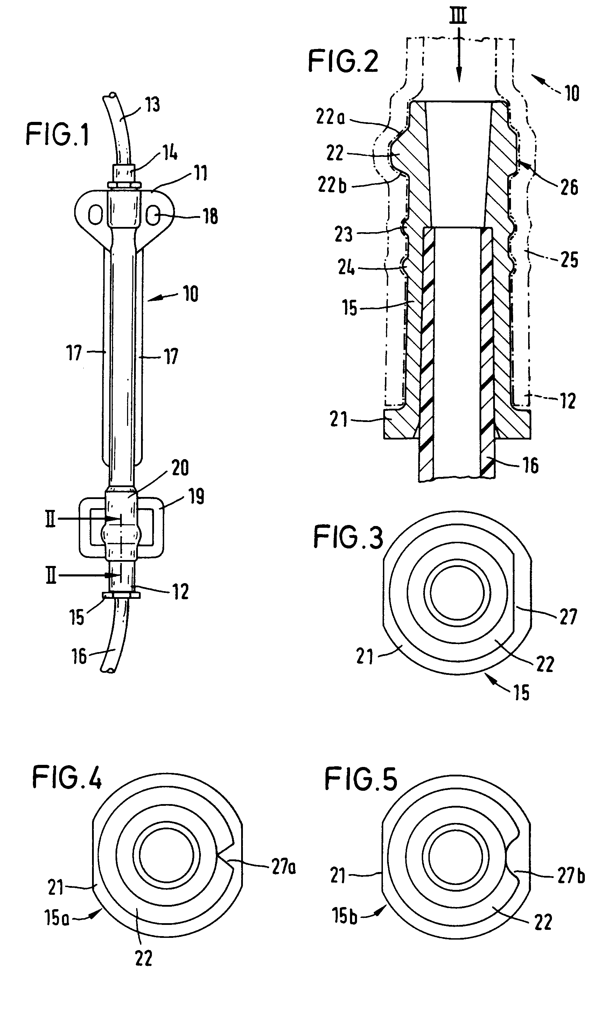

Der in Fig. 1 dargestellte Schlauchset weist einen Pumpenschlauch 10 aus weichelastischem Kunststoff, z.B. Silikonmaterial, auf. Dieser Pumpenschlauch 10 kann in eine peristaltische Pumpe (Rollenpumpe oder Fingerpumpe) eingesetzt werden, die den Pumpenschlauch über seine Länge fortlaufend abquetscht und dadurch Flüssigkeit von dem Einlaßende 11 zum Druckende 12 vortreibt. Das Einlaßende 11 des Pumpenschlauchs 10 ist mit einem flexiblen Anschlußschlauch 13 verbunden, der an ein Infusionsgefäß, in dem eine Infusionslösung enthalten ist, angeschlossen werden kann. Zu diesem Zweck ist das Ende des Anschlußschlauchs 13 mit einem Anschlußstück 14 aus relativ hartem Material versehen, das in das Einlaßende 11 des Pumpenschlauchs 10 eingesteckt werden kann und durch Reibung im Pumpenschlauch festgehalten wird.

In das druckseitige Ende 12 des Pumpenschlauchs 10 ist ebenfalls ein relativ hartes oder starres Anschlußstück 15 eingesteckt, das mit einem zum Patienten führenden Anschlußschlauch 16 verbunden ist.

Der Pumpenschlauch 10 ist generell in der Weise ausgebildet wie der Pumpenschlauch nach DE 39 09 657 A1. Das Schlauchlumen hat im wesentlichen ovalen Querschnitt, wobei nach entgegengesetzten Seiten Stege 17 abstehen. An den Enden des Pumpenschlauchs 10 sind Fixierstücke 18 und 19 einstückig angeformt, mit denen der Pumpenschlauch in der peristaltischen Pumpe lagegerecht fixiert werden kann. In der Nähe des druckseitigen Endes 12 befindet sich ein Längenabschnitt 20 von verringerter Wandstärke, der als Druckmeßzone benutzt werden kann, an die ein den Innendruck messender Drucksensor angesetzt werden kann.

Das Anschlußstück 15, das in den Fign. 2 und 3 dargestellt ist, besteht aus einem langgestreckten rohrförmigen Körper, der an einem Ende einen nach außen abstehenden Flansch 21 aufweist, gegen den das Ende des auf den Körper aufgeschobenen Pumpenschlauchs 10 stößt. In das flanschseitige Ende des Anschlußstücks 15 ist der Anschlußschlauch 16 eingesetzt und dort z.B. durch Kleben befestigt.

Das Anschlußstück 15 weist in der Nähe desjenigen Endes, das dem Pumpenschlauch 10 zugewandt ist, einen wulstförmigen umlaufenden Ring 22 größeren Durchmessers auf. Dieser Ring 22 ist an seiner dem Ende des Anschlußstücks zugewandten Seite mit einer relativ flachen Schulter 22a versehen, während die entgegengesetzte Schulter 22b des Ringes 22 relativ steil ist. Hierdurch wird das Aufschieben des Endes des Pumpenschlauchs 10 auf das Anschlußstück 15 erleichtert und das Abziehen erschwert.

Mit axialem Abstand von dem Ring 22 sind weitere Ringe 23 und 24 kleineren Durchmessers an dem Anschlußstück 15 vorgesehen, welche nach außen abstehen und umlaufende Ringwülste bilden.

Der Pumpenschlauch 10 ist in seinem druckseitigen Endbereich als Muffe 25 mit erweitertem Innendurchmesser ausgebildet. Diese Muffe 25 wird auf das Anschlußstück 15 aufgeschoben, wobei sich die Innenwand der Muffe 25 eng an die Kontur des Anschlußstücks 15 anschmiegt. Dies liegt daran, daß der Innendurchmesser der Muffe 25 kleiner ist als der kleinste Außendurchmesser des Anschlußstücks 15.

An einer Stelle des Umfangs ist zwischen dem Pumpenschlauch 10 und dem Anschlußstück 15 ein Überdruckventil 26 ausgebildet. Dieses Überdruckventil wird durch eine Aussparung 27 des Ringes 22 an einer Stelle des Umfangs gebildet. Im Bereich dieser Aussparung 27 hat der Ring 22 eine verringerte Höhe oder Dicke. Gemäß Fig. 3 wird die Aussparung 27 durch eine Abflachung oder Sehne des Ringes 22 begrenzt. Im Bereich der Aussparung 27 ist der Anpreßdruck, mit der der Pumpenschlauch gegen den Ring 22 gedrückt wird, kleiner als in dem restlichen Umfangsbereich des Ringes 22. Wenn der Innendruck im Pumpenschlauch 10 beispielsweise den Wert von 2,8 bar übersteigt, wird die Abdichtung des Pumpenschlauchs im Bereich der Aussparung 27 undicht und Flüssigkeit gelangt an den Ringen 23 und 24 vorbei durch das druckseitige Ende 12 nach außen. Dadurch wird verhindert, daß der Innendruck noch weiter ansteigen kann und eine solche Größe erreicht, daß das Verbindungsstück 15 von dem Pumpenschlauch abspringt. Nach Beendigung des Innendrucks schließt das Überdruckventil 26 selbsttätig wieder, so daß dieses Ventil zugleich die Funktion eines Rückschlagventils hat, das nur in einer Richtung (nämlich von innen nach außen) durchlässig ist.

Bei dem Ausführungsbeispiel von Fig. 4 entspricht das Anschlußstück 15a demjenigen des ersten Ausführungsbeispiels, mit Ausnahme der Tatsache, daß die Aussparung 27a im Ring 22 V-förmig ausgebildet ist und sich mit zunehmendem Radius erweitert.

Bei dem Ausführungsbeispiel von Fig. 5 ist die Aussparung 27b im Ring 15b generell trapezförmig, mit abgerundeten Ecken. Sie könnte auch kreisbogenförmig ausgebildet sein.

Bei den Fign. 4 und 5 dringt der Pumpenschlauch 10 nicht bis an den Boden der Aussparung 27a bzw. 27b vor, so daß ein Durchlaß im Bereich der betreffenden Aussparung stets freigehalten wird. Das Überdruckventil wird hier von dem Pumpenschlauch 10 in Verbindung mit den kontinuierlich umlaufenden Ringen 23 und 24 kleineren Durchmessers gebildet, während der Ring 22 größeren Durchmessers nur als Halteorgan, nicht aber als Abdichtungsorgan wirkt.

## Patentansprüche

1. Medizinische Schlauch-Verbindungsvorrichtung zum Verbinden des Pumpenschlauchs (10) einer peristaltischen Pumpe mit einem Anschlußschlauch (16), wobei der Anschlußschlauch (16) ein in den relativ weichen Pumpenschlauch (10) einschiebbares relativ starres Anschlußstück (15) aufweist,
**dadurch gekennzeichnet,**
daß das Anschlußstück (15) und der damit zusammenwirkende Abschnitt des Pumpenschlauchs derart ausgebildet sind, daß sie ein selbstschließendes Überdruckventil (26) bilden, dessen Öffnungsdruck kleiner ist als derjenige Druck, bei dem sich der Pumpenschlauch (10) und das Anschlußstück (15) voneinander lösen.

2. Medizinische Schlauch-Verbindungsvorrichtung nach Anspruch 1, dadurch gekennzeichnet, daß das Anschlußstück (15) einen Ring (22) aufweist, der den größten Durchmesser des Anschlußstücks bildet und daß dieser Ring (22) mit dem mit ihm zusammenwirkenden Abschnitt des Pumpenschlauchs (10) eine Stelle geringeren Anpreßdrucks bildet.

3. Medizinische Schlauch-Verbindungsvorrichtung nach Anspruch 2, dadurch gekennzeichnet, daß der Ring (22) an seinem Umfang eine Aussparung (27;27a;27b) aufweist.

4. Medizinische Schlauch-Verbindungsvorrichtung nach Anspruch 3, dadurch gekennzeichnet, daß die Breite der Aussparung (27a,27b) sich radial nach außen erweitert.

5. Medizinische Schlauch-Verbindungsvorrichtung nach Anspruch 3, dadurch gekennzeichnet, daß die Aussparung (27) aus einer Abflachung des Ringes (22) besteht.

6. Medizinische Schlauch-Verbindungsvorrichtung nach einem der Ansprüche 1 bis 5, dadurch gekennzeichnet, daß der Öffnungsdruck des Überdruckventils (26) zwischen 2 und 3 bar beträgt.
